(19) **Europäisches Patentamt / European Patent Office / Office européen des brevets**

(11) **EP 3 967 293 A1**

(12) **EUROPEAN PATENT APPLICATION**

(43) Date of publication:
**16.03.2022 Bulletin 2022/11**

(21) Application number: **20214118.0**

(22) Date of filing: **15.12.2020**

(51) International Patent Classification (IPC):
*A61K 8/67* *(2006.01)*    *A61Q 19/08* *(2006.01)*
*A61K 8/37* *(2006.01)*    *A61P 17/02* *(2006.01)*
*A61Q 17/02* *(2006.01)*    *A61K 31/355* *(2006.01)*
*A61K 31/215* *(2006.01)*    *A61K 31/265* *(2006.01)*
*A61K 31/08* *(2006.01)*    *A61K 47/08* *(2006.01)*
*A61K 47/12* *(2006.01)*    *A61K 47/14* *(2017.01)*
*A61K 8/36* *(2006.01)*

(52) Cooperative Patent Classification (CPC):
(C-Sets available)
**A61K 8/678; A01N 49/00; A61K 8/361; A61K 8/37;
A61K 8/375; A61K 9/0014; A61K 31/355;
A61K 47/08; A61K 47/12; A61K 47/14;
A61P 17/02; A61Q 17/02;** A61K 2800/31;
A61Q 19/08                                    (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **09.09.2020   IT 202000021367**

(71) Applicant: **Sigla S.r.l.**
**29121 Piacenza (IT)**

(72) Inventor: **Facchini, Gabriele**
**29121 Piacenza (IT)**

(74) Representative: **Fisauli, Beatrice A. M.**
**Con Lor S.p.A**
**Via Bronzino, 8**
**20133 Milano (IT)**

(54)     **COMPOSITION BASED ON VITAMIN E FOR TOPIC USE**

(57)     The invention relates to the topical use of an anhydrous and biodegradable composition comprising natural vitamin E and/or its derivatives and/or mixtures thereof, and a carrier component V, anhydrous and lipophilic.

The composition is indicated for the repair and regeneration of tissues such as the cutaneous and subcutaneous tissues.

EP 3 967 293 A1

(52) Cooperative Patent Classification (CPC): (Cont.)

C-Sets
**A01N 49/00, A01N 25/08**

## Description

The present invention relates to a composition based on vitamin E and/or its derivatives, and one or more oil-based carriers, in which said components are of completely natural origin, for topical use both in humans and also for veterinary uses.

More specifically, the composition according to the present invention is indicated to be used for non-therapeutic dermo-cosmetic and veterinary purposes, in particular for use as a tissue repair and regeneration treatment in tissues such as the skin and subcutaneous layer. More specifically, said composition is useful for the treatment of lesions in said tissues, in particular lesions such as abrasions, cuts and grazes and wounds, to accelerate the natural cicatrising process in said tissues.

It is known that vitamin E (or tocopherol), in particular that obtained from plant raw materials, has beneficial effects on the skin, thanks to its action mainly as an antioxidant and to combat the propagation of free radicals in the tissues. It is thought that vitamin E is able to reduce the oxidation of the polyunsaturated fatty acids present in the tissues. Following this, the vitamin E helps to preserve the original appearance and consistency of the skin and, therefore, combats ageing.

Vitamin E, whether synthetic or of plant origin, takes the form of a liquid, but its topical use in a pure form is rendered difficult by its consistency; it is, in fact, an extremely viscous liquid, difficult to apply and hard for the skin to absorb.

To facilitate distribution of the vitamin E on the skin and its absorption, the use of special lipophilic solvents has been known for some time, these being used as carrier agents and agents for distribution on the skin.

Initially, as a carrier agent for a preparation with fat-soluble active principles, such as vitamin E, olive oil was used, which, due to its intrinsic properties, also has beneficial effects for the skin. However, the use of this oil was shown to be impractical, mainly because, after application of the preparation on the skin, the skin remained oily for a certain period of time, that is to say until complete absorption of the oil. The duration of the oiliness therefore lengthens the application time of the preparation on the skin and, in the present day, this is considered to be a big problem.

Furthermore, another problem, for certain skin types, for example oily skin, is that there are contraindications to applying preparations containing large amounts of fatty substances.

Currently, as a replacement for olive oil and for its same purpose, silicone oils are frequently used, of which there are many useful varieties, such as cyclomethicones D4, D5, etc., which differ from each other in certain properties, for example viscosity. These oils diffuse without difficulty and, what is more, do not show the problems mentioned above; in particular, high volatility silicone oils remain on the skin for a brief period of time, and are therefore considered to be "light". In addition, these oils are chemically inert, which makes them particularly suitable as dispersing carriers as they do not interact with the component they are carrying, for example an active principle.

However, protracted use of these oils creates problems.

Firstly, frequent treatment of the same area of skin with products containing these oils is harmful in the long term, as these oils have the effect of blocking the skin's pores, causing dryness.

Furthermore, these oils are not biodegradable, which represents a considerable and serious problem. Following recent increases in environmental awareness, it has been realised that the lack of biodegradability of these oils involves, over time, their accumulation in the environment, land and sea, causing pollution and, therefore, harm to the entire planetary ecosystem.

It has now surprisingly been found that a composition as described hereafter comprising among other things natural vitamin E, known fat-soluble substance, also shows good cell regeneration properties if applied to skin lesions such as abrasions, cuts and grazes, wounds and the like, in order to accelerate the natural cicatrisation process in tissues, such as the skin and subcutaneous layer.

The composition of the present invention also has various advantages with respect to compositions of the prior art in which olive oil or silicone oil are used as carrier agent for an extremely viscous substance such as vitamin E.

The composition of the present invention has the advantage of showing good diffusion and carrier properties on the skin, and, substantially, of not causing oiliness because the carrier component of the vitamin E and/or its derivatives is made up of specific oily compounds, all typically of plant origin, instead of the above-mentioned oils.

Furthermore, the composition of the present invention also has an addition property that is particularly useful and advantageous when the composition of the present invention is applied for veterinary purposes, in particular to treat animals, for example, livestock, working animals, pets.

This composition actually also has a repellent action against certain insects.

The term "insect/s" used here is to be understood broadly, unless otherwise indicated, that is to say it refers to small invertebrates belonging both to the class arthropod and, mainly, to the class insects. Among the insects proper it refers, in particular, to mites, fleas, flies (for example Musca domestica), etc.

It is known that insects are attracted by damaged skin and, therefore, if they can, will land on it. It is also known that insects can be carriers of pathogenic agents that are deposited on injuries to the skin and/or inoculated into the host organism through this means. Said pathogenic agents may have even extremely severe consequences for the health of said host organism, for example following infection of the wounds or their introduction into the blood supply, subsequently resulting in systemic diseases caused by them.

This problem is particularly significant and felt in certain areas, for example for human beings living in hot climates and for the owners of animals, as, often, the lesions involved in these problems are exposed, that is to say not protected by clothing, but also not protected by plasters, bandages, etc.

To prevent such an event from occurring is therefore highly desirable to prevent the health of a human being and/or an animal from being endangered, as the sickness or death of the latter may involve even significant economic loss and/or emotional loss for the owner of the animal.

An advantage of the composition according to the present invention is that it does not cause irritation or burning sensations in the skin if applied on skin lesions, as is the case for insect-repellent compounds known to the art.

In addition, the composition according to the present invention has a long-lasting insect-repellent action, in fact it continues for long periods of time, up to 8 hours. This gives a dual advantage, as its infrequent application on the interested area to keep insects away means an economic saving both in terms of lower consumption of the composition and labour, necessary to apply the composition to the animal.

A further advantage offered by the composition according to the present invention relates to the fact that said composition does not emit an odour that is unpleasant either for the animal on which said composition is having the insect-repellent action, or for the person dealing with that animal, so, unlike what occurs with Neem oil (or Azacirachta Indica) which causes annoyance both for the horse and for the person who is dealing with the horse treated with said oil.

Furthermore, as the vitamin E and/or its derivatives used in said composition are of totally natural origin, more specifically of plant origin, and as the carrier component is also of completely plant origin, the composition as a whole has the intrinsic advantage of being completely plant-based and, therefore, biodegradable and ecologically sustainable.

Within this invention, the expression "natural origin" is understood to mean that the chemical component has not been obtained by synthesis in a laboratory, whether industrial or other.

The term "plant origin", on the other hand, is understood to mean that the chemical component derives from plant substances subjected to treatments such as extraction, distillation, pressing, fractionation, purification, concentration or fermentation.

Furthermore, advantageously, said composition is absorbed rapidly by the skin and, in the specific case of the coat of an animal, after treatment with the composition of the present invention, the coat dries out in a short space of time. Finally, as a further advantage, the composition of the present invention acts on the coat of an animal with an overall protective action, as it helps maintain a good level of hydration in the fur and, in addition to this, improves the appearance of the coat which appears more shiny.

The object of the present invention is therefore an anhydrous composition C suitable for topical administration for use in cutaneous and subcutaneous tissue repair and regeneration, said composition comprising (percentages by weight):

I) 8-30%, preferably 8.5-25%, more preferably 8.5-23%, even more preferably 9-20%, of a component selected from vitamin E in the form of R,R,R d-$\alpha$-tocopherol isomer, its derivatives selected from its esters with organic acids $C_2$-$C_8$, and mixtures thereof, and

II) 70 -92%, 70-92%, preferably 75-91.5%, more preferably 77-91.5%, even more preferably 80-91%, of carrier component V, anhydrous and lipophilic.

Said component V is, for preference, selected from one or more of the following lipophilic compounds:

a) ester of an organic acid $C_8$-$C_{18}$ or of carbonic acid with an alcohol selected from:

i) monohydroxylic aliphatic alcohol $C_4$-$C_{26}$, preferably $C_6$-$C_{24}$, and
ii) polyhydroxylic aliphatic alcohol $C_1$-$C_6$, preferably with two or three hydroxyl groups, such as 1,2-propandiol, glycerol;

b) symmetrical or mixed ethers of saturated fatty alcohols $C_6$-$C_{18}$, straight or branched.

Generally, said saturated fatty alcohols $C_6$-$C_{18}$ are primary alcohols, such as octanol-1, decanol-1, hexadecanol-1.

Said aliphatic alcohol may be straight or branched, saturated or unsaturated. Said organic acid is preferably selected from caprylic, capric, palmitic, stearic, lauric, adipic, myristic acids or a mixture thereof.

For preference, said monohydroxylic alcohol has the formula R-OH, in which R is an alkyl radical $C_4$-$C_{26}$.

More specifically, said compounds are selected, for example, from dicaprylyl ether, 2-propyl-heptyl caprylate, caprylyl caprylate/caprate, dicaprylyl carbonate, coco caprylate, coco caprylate/caprate, propylene glycol dicaprylate/dicaprate, 2-ethyl-hexyl palmitate, 2-ethyl-hexyl stearate, hexyl laurate, dibutyl adipate, isopropryl palmitate, isopropryl myristate, cetearyl 2-ethyl hexanoate.

As is known to the art, the following above-mentioned terms are used to designate the following mixtures of esters:

- "caprylyl caprylate/caprate": mixture of esters of caprylic and capric acids with caprylic alcohol;
- "coco caprylate": mixture of esters of capric acid with coconut fatty alcohols;
- "coco caprylate/caprate": mixture of esters of caprylic and capric acids with coconut fatty alcohols; and
- "propylene glycol dicaprylate/dicaprate": a mixture of diesters of propylene glycol with caprylic acid and capric acid.

Naturally, the component V may also consist of two or more of said compounds. In that case, said compounds may be present in any ratio by weight.

In the composition according to the present invention, component V is typically of natural origin, preferably plant. Said component V is totally biodegradable. The natural origin index of component V is defined under guideline ISO 16128. In general, said component V is for preference a compound having a diffusion index ("spreading value") comprised between 700 and 2000, preferably between 700 and 1800, $mm^2$/10 min. and a viscosity comprised between 1 and 21 mPa·s, measured at 20°C.

As mentioned previously, component V acts as a carrier means for the additional components present in composition C. As well as this function, component V generally also has other actions, such as, for example, moisturising, soothing, nourishing, softening, protecting.

If component B is an ester, this is preferably one without smell or with only a slight fragrance.

Component V is produced using procedures known to an expert in the field, so these will not be described in the present description.

According to a preferred embodiment, in the composition according to the present invention component V is in the form of an ester, such as caprylyl caprylate/caprate, it is for the most part, that is to say at least 75%, preferably at least 80%, more preferably at least 85%, by weight, or even entirely, produced by means of an eco-sustainable procedure, such as a fermentation process. Thus, typically and preferably, only a reduced fraction of said component V in the form of an ester, that is to say 25% or less, generally between 0.5 and 20%, in particular between 1 and 15%, preferably between 1 and 10%, by weight, is produced using a procedure that does not implement fermentation.

Said fermentation procedure, implemented according to known technology, comprises the use of micro-organisms and/or enzymes. The latter derive, preferably, from natural organisms, such as plants and yeasts. A first advantage of the procedures involving the use of enzymes is, in this specific case, the efficiency of these methods as a result of the high selectivity of the enzymes that allow very uniform production, that is to say with a very low rate of sub-products and, therefore, a high yield. Sub-products also refer to a product with a different stereospecificity from the one required.

Fermentation procedures also have the advantage of producing less processing waste compared to traditional procedures, as the micro-organisms and/or enzymes can be used repeatedly in the subsequent procedures to obtain the same types of product, so that the environmental impact is reduced with respect to a conventional process, in which the chemical catalysts, which are used in place of the enzymes, are generally eliminated after having been used once only.

Preferably, the enzymes that are used as biocatalysts in the procedures to produce said component V are certified as produced by non-genetically modified micro-organisms.

In the composition according to the present invention, as mentioned previously, the vitamin E (or tocopherol) is of natural origin, so the vitamin E is only present as a R,R,R-α-tocopherol isomer, also known as d-α-tocopherol or 2R,4'R,8'R-α-tocopherol, instead of in the form of a raceme.

In the present description, unless specified, "vitamin E2 is intended to refer both to vitamin E in its free form and its derivatives and mixtures thereof.

It is known that vitamin E and its derivatives obtained from natural plant sources have a much higher bioavailability than vitamin E and its derivatives of synthetic origin.

Should the composition C according to the present invention contain two or more compounds that can be traced to vitamin E, said compounds may be present in any ratio by weight.

As mentioned previously, vitamin E in the form of an ester is esterified with an organic acid, generally, with a low number of carbon atoms, typically $C_2$-$C_8$, typically mono- or dicarboxylic, generally saturated, generally acetic or succinic acid.

The vitamin E, and its said derivatives, used in the composition of the present invention can be obtained, typically, by distillation of plant oils.

Optionally, the composition C may comprise, in addition to the above-mentioned components, at least an additional component to be carried, indicated in the following as component (III). Naturally, said component (III) is likewise of natural origin, preferably of plant origin, in any case totally biodegradable.

In particular, said additional component to be carried is, preferably, at least in part fat-soluble, more preferably it is totally fat-soluble. This component is selected, for preference, from those capable of being in synergy with the activity of vitamin E and/or its derivatives, such as, merely as an example, polyunsaturated fatty acids, in particular one or more of ω-3-, ω-6- and ω-9-polyunsaturated $C_{10}$-$C_{22}$ fatty acids and the like, and mixtures thereof.

Suitable ω-3-polyunsaturated fatty acids are, for example, eicosapentaenoic acid (EPA), docosahexaenoic acid (DHA), docosapentaenoic acid (DPA) and alpha-linolenic acid (ALA).

Examples of further suitable fatty acids, in particular ω-6- and/or ω-9-polyunsaturated, are linoleic acid, γ-linolenic acid, arachidonic acid and docosadienoic acid, to mention just a few.

If present, said additional component (III) is in an amount between 0.5 and 20%, preferably between 1 and 15%, more preferably between 2 and 10%, by weight with respect to the entire weight of composition C.

Naturally, "additional component to be carried" is understood to refer also to two or more active components. In this case, said active components can be in any ratio by weight with each other.

Following the components present in composition C according to the present invention, said composition C is therefore, at least to a great extent, that is to say equal to or exceeding 95%, preferably 100%, by weight, of plant origin and, therefore, totally biodegradable. More specifically, said composition C, like its components (II) and (III), have a natural origin index of between 0.7 and 1, more preferably between 0.9 and 1.

To assess the ecological profile of the composition according to the present invention, the term "natural origin index" is used here to refer to the percentage by weight of the component directly obtained from natural sources or obtained from immediate predecessors that can be obtained directly from natural sources.

According to current regulations, an ingredient with an index of naturalness equal to 1 is considered natural, whereas an ingredient with an index of naturalness of 0.5 or above, but less than 1 is a natural derivative.

In certain embodiments, the composition described may include at least one component with an index of naturalness less than 1.

Composition C according to the present invention, therefore, is essentially free from alkane type hydrocarbons in free form.

Composition C according to the present invention can be prepared, typically, using a simple preparation process. This process is essentially a process mixing the two or more components that make it up, using known methods that, consequently, are known to an expert in the field.

As the preparation methods are well known to an expert in the field, they will not be described in detail.

Particular operating conditions, such as temperature, mixing speed, etc., are not crucial to carry out the preparation process. This process is based, essentially, on an operation to mix the components, preferably in a liquid state. Indicatively, the temperature at which mixing takes place may vary between 20° and 60° C. Generally the component V is in liquid or paste form at room temperature.

The composition C according to the present invention is suitable for topical use. In particular the composition is suitable for application to tissues such as the skin and subcutaneous layer to encourage cicatrisation.

Said composition is also suitable for applications on the skin and hair to improve moisturising, as mentioned above, and also for application on the vaginal and perianal mucosa. In particular, the composition is suitable, for example, in the case of dermatitis, such as atopical dermatitis, contact dermatitis, dermatitis herpetiformis, seborrhoeic dermatitis, stress dermatitis, perioral dermatitis, sweat rash, and psoriasis.

Composition C can be formulated in various ways, both based on the individual on whom it is to be used (human being or non-human animal), and the application method.

Consequently, according to this, composition C may contain additional components that are appropriate to and usually used for the required formulation (such as gelling agent for fats, waxes or butters of plant origin, etc.). Composition C may be formulated in such a way as to appear, for example, as a spray, gel, ointment, liquid.

According to the type of formulation, composition C according to the present invention will be suitable for a specific container, of a larger or smaller size according to whether the composition is to be used for human or veterinary purposes. These containers are, for example, BOV or pump spray, tube, bottle, jar, etc., preferably preferring highly eco-sustainable materials.

The following is a non-limiting example illustrating the present invention.

Materials and methods

The materials used to prepare the examples provided have the properties indicated in table A below.

Table A

| Component V | Natural origin index | Index of diffusion mm$^2$/10 min. | Viscosity mPa·s (at 20°C) |
|---|---|---|---|
| Dicaprylyl ether | 1 | 1600 | 4 |

(continued)

| Component V | Natural origin index | Index of diffusion mm$^2$/10 min. | Viscosity mPa·s (at 20°C) |
|---|---|---|---|
| caprylyl caprylate/caprate | 1 | 1400 | 5 |
| Coco caprylate/caprate | 1 | 800 | 11 |

The viscosity is measured using a Rheomat RM 180 rheometer. The sample analysed has a temperature between 20-25°C. The settings for the viscosity test are: bob 9, tube 1, 1500 shear rate for 5 min. (300 sec.).

The index of diffusion of a compound is defined as the area (in mm$^2$) of a skin surface covered by said compound in a specific period of time.

The method used to determine the index of diffusion of a liquid component is an *in vivo* test. This method consists in applying 4 mg of the component to be analysed on the front forearm of an individual. For each component, 4 separate applications were carried out on the same individual. The test is carried out at a temperature of 23°C and at a relative humidity of 60%. Ten minutes after application of a sample on the forearm the area of diffusion was photographed and measured.

The natural origin index is defined under guideline ISO 16128.

The caprylyl caprylate/caprate is produced using a fermentation procedure., unlike coco caprylate/caprate and dicaprylyl ether which are entirely produced using classical chemical methods.

Calculation of the percentage repair of a wound

The average value of the width of a wound at each point in time, and therefore the percentage repair of the wound, is calculated using the formula:

$$\% \text{ wound reduction} = 100 - (\text{average wound width at time X} / \text{average wound width at } T_0) \times 100$$

The percentage repair of the wound is compared with that of the negative control to judge the effectiveness.

Calculation of the percentage protection

The percentage protection (PP) indicates the reduction of the number of flies on the substrate (that is to say the food mixture) treated with respect to the untreated substrate, and it is calculate using the following formula:

$$\% \text{ PP} = 100 - [(\text{n}° \text{ of flies on the treated mixture} / \text{n}° \text{ of flies on the untreated mixture}) \times 100]$$

The data is analysed using a mixed linear model, which is a test used to compare pairs of tests to assess whether there is a significant difference, and a t test for paired samples, which is a statistical procedure used to determine whether the average difference between two sets of observations is zero. In the t test for paired samples the difference between the sample examined and the control is assessed at each point in time.

Examples

Examples 1-4

The component to be carried is added to the carrier component, an oily liquid, subjected to stirring, and then stirring is continued for 30 minutes, until the components are completely mixed.

Table 1 below shows the components and the quantities (percentages by weight) for each composition produced.

In the following tables, the natural form of vitamin E is indicated with "d".

Table 1

| Example | Component to be carried | | Carrier component | |
|---|---|---|---|---|
| | type | % | type | % |
| 1 | d-alpha tocopheryl acetate | 15 | caprylyl caprylate/caprate | 85 |
| 2 | d-alpha tocopheryl acetate | 10 | caprylyl caprylate/caprate | 90 |
| 3 | d-alpha tocopheryl acetate | 15 | dicaprylyl ether | 85 |
| 4 | d-alpha tocopherol | 10 | dicaprylyl ether | 90 |

The compositions obtained in this way are all easily spreadable, quickly absorbed and not oily.

Examples 5 and 6

Example 1 is repeated, with the difference that the carrier component is made up of two different components.
Table 2 below shows the components and the quantities (percentages by weight) for each of them.

Table 2

| Example | Component to be carried | | Carrier component | |
|---|---|---|---|---|
| | type | % | type | % |
| 5 | d-alpha tocopheryl acetate | 15 | caprylyl caprylate/caprate | 80 |
| | | | coco caprylate/caprate | 5 |
| 6 | d-alpha tocopherol | 15 | caprylyl caprylate/caprate | 75 |
| | | | coco caprylate/caprate | 10 |

The compositions obtained in this way are all easily spreadable, quickly absorbed and not oily.

Example 7

Example 1 is repeated, with the difference that mixing is carried out in the presence of an additional component to be carried (III), different from tocopherol, as an additional component in the final composition.
Table 3 below shows the components and the quantities (percentages by weight) for each of them.

Table 3

| Example | Components to be carried | | Carrier component | |
|---|---|---|---|---|
| | type | % | type | % |
| 7 | d-alpha tocopheryl acetate | 10 | caprylyl caprylate/caprate | 85 |
| | mixture of $\omega$-3-polyunsaturated fatty acids [1] | 5 | | |
| [1]The mixture consists of the following $\omega$-3-polyunsaturated fatty acids ALA, EPA, DHA, present in the following ratios (by weight):1:3:2. | | | | |

The compositions obtained in this way also have nourishing properties and are all easily spreadable, quickly absorbed and not oily.

Example 8

Example 7 is repeated, with the difference that mixing is carried out in the presence of the carrier component made up of two different components (III).
Table 4 below shows the components and the quantities (percentages by weight) for each of them.

Table 4

| Example | Components to be carried | | Carrier component | |
|---|---|---|---|---|
| | type | % | type | % |
| 8 | d-alpha tocopheryl acetate | 10 | caprylyl caprylate/caprate | 75 |
| | mixture of ω-3-polyunsaturated fatty acids[1] | 5 | coco caprylate/caprate | 10 |
| [1]The mixture consists of the following ω-3-polyunsaturated fatty acids ALA, EPA, DHA, present in the following ratios (by weight):1:3:2. | | | | |

The compositions obtained in this way are all easily spreadable, quickly absorbed and not oily.

Example 9

Example 8 is repeated, with the sole difference that in place of the mixture of ω-3-polyunsaturated fatty acids, sweet almond oil is used as component (III). The properties of the composition obtained are similar to those of the composition of example 8.

Examples 1A-9A and control example C1

The compositions of the preceding examples 1-9 were tested *in vitro* in a test to repair an artificial wound, the test measures the migration capacity of the cells.

The cells used were a strain of transformed murine endothelioma brain cells, known as bEnd.3, supplied by the firm SIGMA. These cells simulate the vessel wall and, consequently, are used for *in vitro* study of angiogenesis and the ability of vessel walls to repair.

The cells were cultivated in DMEM containing 10% FBS and antibiotics.

The test was carried out in triplicate. The cells were cultivated in a single layer in 24 well plates. After incubating the cells for 24 hours, a cut was made, using a sterile plastic spike, across the whole length of the cellular single layer obtained, to simulate a wound.

The composition to be tested was dissolved, first, in isopropanol to obtain a solution in which the composition to be tested had a concentration of 200 mg/ml, said solution is diluted in the cell culture medium so as to obtain in said culture medium a concentration of the composition to be tested indicated in table 5.

The cells were cultivated at a temperature of 37°C with 5% $CO_2$ in the presence of the culture medium containing the composition to be tested, for a period of 24h. Cells cultivated in the same conditions indicated above were used as a negative control, with the sole difference that the culture medium did not contain said composition.

At different times during the 24h observations were carried out on the cells using phase contrast microscopy, both on the cells treated with the composition and the control cells, using a Leica DM-IL IMC microscope with an enlargement of between 100 and 200X, with the size of the wound being recorded in $\mu$m. The images are documented using a digital video camera and the image software ISCapture.

The extent of the cicatrisation was assessed as the average reduction in width of the wound over time, during the first 24 hours after treatment with a dose of 1 mg/ml of the preparation according to the present invention compared to the average width of the cut at time To. The results of the test are shown in table 5 below.

Table 5

| Example | Quantity of component to be carried in composition C (%) | Cicatrisation | | |
|---|---|---|---|---|
| | | 3 hours | 6 hours | 24 hours |
| 1A | 15 | 5.10 | 11.67 | 50.74 |
| 2A | 10 | 5.01 | 11.05 | 50.03 |
| 3A | 15 | 5.08 | 11.65 | 50.73 |
| 4A | 10 | 4.90 | 10.95 | 50.01 |
| 5A | 15 | 5.11 | 11.69 | 50.76 |
| 6A | 15 | 5.10 | 11.67 | 50.74 |

(continued)

| Example | Quantity of component to be carried in composition C (%) | Cicatrisation | | |
|---|---|---|---|---|
| | | 3 hours | 6 hours | 24 hours |
| 7A | 15 | 5.05 | 11.50 | 51.60 |
| 8A | 15 | 5.04 | 11.45 | 51.58 |
| 9A | 15 | 5.01 | 11.30 | 50.95 |
| C1 | 0 | 1.33 | 13.28 | 41.89 |

The percentage data for the reduction in width of a wound shown in table 5 above demonstrate that composition C has an effective action in reducing cuts compared with a wound that is not treated with the composition of this invention.

Example 10

The composition of example 3 above was formulated as an aerosol spray and used in that form in a test to verify its repellent action on flies *(Musca domestica)*.

Four tests were carried out with said composition, using flies from the Entostudio laboratory raised in cubic cages with sides measuring 50 cm, at $25 \pm 1$ °C and $50 \pm 5\%$ relative humidity. Exposure to the light was for 12 hours a day (24 hours) and the light intensity was 300 lux at 6000°K.

In each test a recipient was used (capacity 65 l and size 56x39x42 cm) made of transparent plastic, with a top aperture completely covered by a metal mesh. The recipients were kept in a room with walls, ceiling and floor coloured completely white.

On the flat bottom of each recipient were a cup (T1) and a control cup (Tc). Both cups (diameter 12 cm and height 6 cm) are made of transparent plastic and each contain a food mixture consisting of 50 g of bran mixed with powdered milk and water. Cup T1 contains said food mixture on which the composition of example 3 has been sprayed in the form of an aerosol for a time of 2 seconds and at a distance of 20 cm. The amount of composition sprayed on each cup is indicated in table 6 below.

Into each of the four recipients 50 flies were introduced, and, from the time of their introduction, at regular time intervals for a period of 6 hours the flies that landed on each of the two cups T1 and Tc were counted.

After 6 hours a total of 13 flies had landed on cup T1 and 289 flies on cup Tc.

Comparison examples C2 and C3

Example A is repeated with the sole difference that neither of the two cups per recipient contains the treated mixture.

Table 6 below indicates the data for the number of flies counted at each point in time, relating both to example A and to comparison examples 1c and 2c.

The data indicated in the table demonstrate that composition C according to the present invention has an evident repellent effect on flies.

The compositions of examples 1, 2 and 4-9 above have also shown, with data not reported here, that they have an insect-repellent effect on flies similar to that of composition 3.

Table 6

| Examples | Grams released in 2" | Detection time | | | | | | | | | | | |
|---|---|---|---|---|---|---|---|---|---|---|---|---|---|
| | | 0.5 h | | 1h | | 1.5h | | 2h | | 2.5h | | 3h | |
| | | T1 | Tc | T1 | Tc | T1 | Tc | T1 | Tc | T1 | Tc | T1 | Tc |
| 1 | 2.13 | 1 | 2 | 1 | 1 | 2 | 1 | 0 | 2 | 1 | 4 | 1 | 9 |
| 2 | 2.08 | 0 | 4 | 0 | 1 | 0 | 5 | 0 | 7 | 1 | 10 | 0 | 14 |
| 3 | 2.11 | 0 | 1 | 0 | 1 | 0 | 3 | 0 | 6 | 0 | 6 | 0 | 7 |
| 4 | 2.05 | 1 | 2 | 0 | 2 | 1 | 2 | 0 | 5 | 1 | 6 | 0 | 10 |
| Average examples 1-4 (%) | 2.09 | 1.00 | 4.50 | 0.50 | 2.50 | 1.50 | 5.50 | 0.00 | 10.00 | 1.50 | 13.00 | 0.50 | 20.00 |
| 2c | 0 | 3 | 0 | 3 | 2 | 4 | 3 | 4 | 5 | 9 | 6 | 8 | 2 |
| 3c | 0 | 2 | 2 | 3 | 1 | 5 | 4 | 3 | 4 | 10 | 7 | 7 | 3 |
| Average examples 2c-3c (%) | 0 | 5.00 | 2.00 | 6.00 | 3.00 | 9.00 | 7.00 | 7.00 | 9.00 | 19.00 | 13.00 | 15.00 | 5.00 |

Table 6 - continued

| Examples | Grams released in 2" | Detection time | | | | | | | | | | | |
|---|---|---|---|---|---|---|---|---|---|---|---|---|---|
| | | 3.5 h | | 4h | | 5h | | 6h | | 7h | | 8h | |
| | | T1 | Tc | T1 | Tc | T1 | Tc | T1 | Tc | T1 | Tc | T1 | Tc |
| 1 | 2.13 | 1 | 2 | 0 | 5 | 0 | 5 | 0 | 2 | 0 | 3 | 0 | 4 |
| 2 | 2.08 | 0 | 4 | 1 | 12 | 0 | 12 | 0 | 8 | 0 | 14 | 0 | 9 |
| 3 | 2.11 | 0 | 1 | 0 | 7 | 0 | 10 | 0 | 6 | 0 | 5 | 0 | 13 |
| 4 | 2.05 | 1 | 2 | 1 | 8 | 0 | 10 | 0 | 5 | 0 | 7 | 0 | 9 |
| Average examples 1-4 (%) | 2.09 | 0.50 | 12.00 | 1 | 16.00 | 0 | 18.50 | 0 | 10.50 | 0 | 14.50 | 0 | 17.50 |
| 2c | 0 | 4 | 0 | 5 | 9 | 6 | 3 | 4 | 3 | 5 | 4 | 8 | 3 |
| 3c | 0 | 4 | 2 | 4 | 10 | 5 | 5 | 3 | 4 | 6 | 5 | 6 | 5 |
| Average examples 2c-3c (%) | 0 | 8.00 | 2.00 | 9.00 | 19.00 | 11.00 | 8.00 | 7.00 | 7.00 | 11.00 | 9.00 | 14.00 | 8.00 |

**Claims**

1. An anhydrous composition C suitable for topical administration for use in cutaneous and subcutaneous tissue repair and regeneration, said composition C comprising (percentages by weight):

   I) 8-30% of a component selected from vitamin E in the form of R,R,R-d-α-tocopherol isomer, its derivatives selected from its esters of an organic acid $C_2$-$C_8$, and mixtures thereof, and

   II) 70-92% of a carrier component V, anhydrous, selected from one or more of the following lipophilic compounds:

   a) ester of an organic acid $C_8$-$C_{18}$ or of carbonic acid with an alcohol selected from:

   i) monohydroxylic aliphatic alcohol $C_4$-$C_{26}$, preferably $C_6$-$C24$, and
   ii) polyhydroxylic aliphatic alcohol $C_1$-$C_6$, preferably with two or three hydroxyl groups;

said organic acid being selected from caprylic, capric, palmitic, stearic, lauric, adipic, myristic acids or a mixture thereof; and

b) symmetrical or mixed ethers of saturated fatty alcohols $C_6$-$C_{18}$, straight or branched.

2. Composition C for use according to the preceding claim, comprising (percentages by weight):

- 8.5-25% of vitamin E or derivatives thereof or their mixtures, and
- 75-91.5% of component V.

3. Composition C for use according to any one of the preceding claims, comprising (percentages by weight):

- 8.5-23% of vitamin E or derivatives thereof or their mixtures, and
- 77-91.5% of component V.

4. Composition C for use according to any one of the preceding claims, in which component V is selected from dicaprylyl ether, propyl heptyl caprylate, caprylyl caprylate/caprate, dicaprylyl carbonate, coco caprylate, coco caprylate/caprate, propylene glycol dicaprylate dicaprate, ethylhexyl palmitate, ethylhexyl stearate, hexyl laurate, dibutyl adipate, isopropryl palmitate or myristate, cetearyl ethyl hexanoate and/or a mixture thereof.

5. Composition C for use according to any one of the preceding claims, comprising a component (III) in a quantity of from 0.5 to 20% by weight with respect to the entire weight of composition C, said component (III) being selected from polyunsaturated fatty acids selected from fatty acids ω-3-, ω-6- and ω-9-polyunsaturated $C_{10}$-$C_{22}$ or mixtures thereof.

6. Composition C for use according to the preceding claim in which component (III) is in an amount of between 1 and 15% by weight with respect to the entire weight of composition C.

7. Composition C for use according to the preceding claim in which component (III) is in an amount of between 2 and 10% by weight with respect to the entire weight of composition C.

8. Composition C for use according to any one of the preceding claims, **characterised in that** it is in the form of a spray, a cream, a gel, a lotion, a serum, a foam or an ointment.

9. Composition C for use according to any one of the preceding claims, **characterised in that** it is for human and/or veterinary use and is used to treat lesions such as abrasions, cuts and grazes and wounds.

10. Composition C for use according to any one of the preceding claims that also acts as an insect-repellent.

Europäisches
Patentamt

European
Patent Office

Office européen
des brevets

# EUROPEAN SEARCH REPORT

Application Number

EP 20 21 4118

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/015018 A1 (ZECCARDO ERMELINDA [IT] ET AL) 18 January 2018 (2018-01-18) * page 3 * ----- | 1-9 | INV. A61K8/67 A61Q19/08 A61K8/37 A61P17/02 A61Q17/02 A61K31/355 A61K31/215 A61K31/265 A61K31/08 A61K47/08 A61K47/12 A61K47/14 A61K8/36 |
| X | DATABASE GNPD [Online] MINTEL; 27 January 2020 (2020-01-27), anonymous: "Natural Spray Against Flies and Mosquitoes", XP55809783, Database accession no. 7214151 | 1,10 | |
| Y | * abstract * ----- | 10 | |
| X | Für: "VITAMIN E ÖL extra stark", , 17 April 2019 (2019-04-17), XP55809791, Retrieved from the Internet: URL:https://api.gebrauchs.info/2b8840bf6b6 bf464c88c07ee6a0b6db2i [retrieved on 2021-06-01] * the whole document * ----- | 1 | |
| X | Anonymous: "Natures Aid Vitamin E Oil 20.000 iu (50 ml)", , 23 January 2018 (2018-01-23), XP55809793, Retrieved from the Internet: URL:https://dolphinfitness.de/de/natures-a id-vitamin-e-oil-20000-iu-50-ml/142621 [retrieved on 2021-06-01] * the whole document * ----- | 1 | TECHNICAL FIELDS SEARCHED (IPC) A61K A61Q A61P |
| X | EP 3 061 446 A1 (BIO LO GA SRL [IT]) 31 August 2016 (2016-08-31) * paragraphs [0019], [0029] - [0033] * * examples 1,2,5-7 * ----- | 1-9 | |

-/--

The present search report has been drawn up for all claims

| Place of search | Date of completion of the search | Examiner |
|---|---|---|
| Munich | 1 June 2021 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 20 21 4118

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP H11 255606 A (SEKISUI CHEMICAL CO LTD) 21 September 1999 (1999-09-21) * abstract * ----- | 10 | |
| X | WO 97/45098 A2 (PANIN GIORGIO [IT]) 4 December 1997 (1997-12-04) * the whole document * ----- | 1 | |

TECHNICAL FIELDS
SEARCHED      (IPC)

The present search report has been drawn up for all claims

| Place of search | Date of completion of the search | Examiner |
|---|---|---|
| Munich | 1 June 2021 | Skulj, Primoz |

EPO FORM 1503 03.82 (P04C01)

**ANNEX TO THE EUROPEAN SEARCH REPORT
ON EUROPEAN PATENT APPLICATION NO.**                    EP 20 21 4118

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2021

| Patent document cited in search report | Publication date | Patent family member(s) | | Publication date |
|---|---|---|---|---|
| US 2018015018 A1 | 18-01-2018 | AR | 103607 A1 | 24-05-2017 |
| | | AU | 2016214625 A1 | 03-08-2017 |
| | | BR | 112017016652 A2 | 10-04-2018 |
| | | CA | 2974852 A1 | 11-08-2016 |
| | | CL | 2017001889 A1 | 13-04-2018 |
| | | CN | 107405302 A | 28-11-2017 |
| | | CY | 1121366 T1 | 29-05-2020 |
| | | DE | 202015100655 U1 | 04-02-2016 |
| | | DK | 3253383 T3 | 18-02-2019 |
| | | EA | 201791332 A1 | 31-01-2018 |
| | | EP | 3253383 A1 | 13-12-2017 |
| | | ES | 2711171 T3 | 30-04-2019 |
| | | HR | P20190328 T1 | 19-04-2019 |
| | | HU | E042413 T2 | 28-06-2019 |
| | | LT | 3253383 T | 25-02-2019 |
| | | PL | 3253383 T3 | 31-05-2019 |
| | | PT | 3253383 T | 30-01-2019 |
| | | SI | 3253383 T1 | 29-03-2019 |
| | | TR | 201901911 T4 | 21-03-2019 |
| | | US | 2018015018 A1 | 18-01-2018 |
| | | WO | 2016124408 A1 | 11-08-2016 |
| EP 3061446 A1 | 31-08-2016 | DK | 3061446 T3 | 04-11-2019 |
| | | EP | 3061446 A1 | 31-08-2016 |
| | | ES | 2759271 T3 | 08-05-2020 |
| | | HR | P20192331 T1 | 20-03-2020 |
| | | HU | E047279 T2 | 28-04-2020 |
| | | LT | 3061446 T | 25-11-2019 |
| | | PL | 3061446 T3 | 31-03-2020 |
| | | PT | 3061446 T | 29-10-2019 |
| | | SI | 3061446 T1 | 28-02-2020 |
| JP H11255606 A | 21-09-1999 | NONE | | |
| WO 9745098 A2 | 04-12-1997 | AT | 251906 T | 15-11-2003 |
| | | AT | 369850 T | 15-09-2007 |
| | | AU | 713816 B2 | 09-12-1999 |
| | | BR | 9709415 A | 11-01-2000 |
| | | CA | 2256403 A1 | 04-12-1997 |
| | | DE | 69725575 T2 | 29-07-2004 |
| | | DE | 69738034 T2 | 15-05-2008 |
| | | EP | 0910367 A2 | 28-04-1999 |
| | | EP | 1342473 A1 | 10-09-2003 |
| | | ES | 2208910 T3 | 16-06-2004 |
| | | ES | 2291559 T3 | 01-03-2008 |
| | | IT | MI961121 A1 | 01-12-1997 |

EPO FORM P0459

For more details about this annex : see Official Journal of the European Patent Office, No. 12/82

page 1 of 2

EP 3 967 293 A1

**ANNEX TO THE EUROPEAN SEARCH REPORT
ON EUROPEAN PATENT APPLICATION NO.**

EP 20 21 4118

This annex lists the patent family members relating to the patent documents cited in  the above-mentioned European search report.
The members are as contained in the European Patent Office EDP file on
The European Patent Office is in no way liable for these particulars which are merely given for the purpose of information.

01-06-2021

| Patent document cited in search report | Publication date | Patent family member(s) | Publication date |
|---|---|---|---|
| | | JP 2000511168 A | 29-08-2000 |
| | | PT 910367 E | 27-02-2004 |
| | | PT 1342473 E | 10-10-2007 |
| | | SI 1342473 T1 | 31-12-2007 |
| | | WO 9745098 A2 | 04-12-1997 |

EPO FORM P0459

For more details about this annex : see Official Journal of the European Patent Office, No. 12/82

page 2 of 2